# EUROPEAN PATENT SPECIFICATION

(11) **EP 3 978 547 B1**
(45) Date of publication and mention of the grant of the patent: **11.12.2024**
(21) Application number: 19932863.4
(22) Date of filing: 29.08.2019
(51) Int. Cl.: C07D 239/545, C08G 73/06, C08L 27/06, C08L 79/04

(54) **METHOD FOR SOLID-PHASE SYNTHESIS OF CYANURIC ACID-6-AMINO-1,3-DIMETHYLURACIL COMPLEX ZINC SALT**
VERFAHREN ZUR FESTPHASENSYNTHESE VON CYANURSÄURE-6-AMINO-1,3-DIMETHYLURACIL-KOMPLEX ZINKSALZ
PROCÉDÉ DE SYNTHÈSE EN PHASE SOLIDE D'UN SEL DE ZINC D'UN COMPLEXE D'ACIDE CYANURIQUE-6-AMINO-1,3-DIMETHYLURACIL

(30) Priority: 13.06.2019 CN 201910509991
(43) Date of publication of application: 06.04.2022
(73) Proprietor: Shenzhen Aimsea Industrial Co., Ltd, Shenzhen, Guangdong, 518118 (CN)
(72) Inventor: YAN, Yifeng, Shenzhen, Guangdong 518118 (CN); ZHOU, Zhixin, Shenzhen, Guangdong 518118 (CN); YAN, Qing, Shenzhen, Guangdong 518118 (CN)
(74) Representative: KIPA AB
(86) International application number: PCT/CN2019/103165
(87) International publication number: WO 2020/248381

(56) References cited:
- CN-A- 102 391 196
- CN-A- 102 391 196
- CN-A- 103 183 644
- CN-A- 106 366 514
- CN-A- 106 432 957
- CN-A- 106 518 795
- CN-A- 106 632 113
- CN-A- 106 633 053
- JP-A- 2012 236 867
- US-A- 4 507 270
- US-A- 5 925 696

## Description

### FIELD OF THE INVENTION

The present application relates to the technical field of a cyanuric acid-uracil complex zinc salt preparation, in particular to a method for a solid-phase synthesis of a cyanuric acid-uracil complex zinc salt.

### BACKGROUND

Polyvinyl chloride (PVC), polyethylene, polypropylene, polystyrene and ABS (Acrylonitrile Butadiene Styrene) are together called as five major thermoplastic synthetic resins. For common used synthetic resins, a yield of PVC is lower than that of polyethylene, ranking second. The PVC has an excellent chemical corrosion resistance, electrical insulation, flame retardancy and mechanical property, together with a plurality of advantages including a light weight, a high strength, a low cost, and more, having been widely used in various fields of industry and agriculture, construction, electrical and electronics, and daily necessities.

Due to an unstable structure existing in a molecular chain period of the PVC, while a processing temperature of the PVC is close to a thermal decomposition temperature thereof, it is easy to generate a degradation due to losing HCl, resulting in discoloration of a product and a decreased performance of usage. Therefore, a stabilizer shall be added during a thermal processing of the PVC.

A plurality of organic thermal stabilizers in the prior art, wherein most own a plurality of functions including absorbing HCl, complexing a metal chloride which is able to catalyze a PVC degradation and more. However, the functions of a thermal stabilizer for the PVC, wherein an ability of replacing an unstable chlorine atom is most critical, since absorbing HCl can only delay the PVC degradation, while replacing the unstable chlorine atom can eliminate a plurality of factors causing the PVC degradation at a first beginning, before preventing the PVC degradation. Thus a primary stabilizer must have an ability of replacing the unstable chlorine atoms in a PVC chain. A Nitrogen-containing organic stabilizer is a class of thermal stabilizer that has the ability of replacing the unstable chlorine atoms. Although the nitrogen-containing thermal stabilizers having been developed have a weaker ability to replace the unstable chlorine atoms, thus cannot be used as the primary stabilizer, it is still a most potential variety among the organic thermal stabilizers being able to develop into the primary stabilizer.

A calcium-zinc thermal stabilizer is a typical representative of a plurality of non-toxic additives in a polyvinyl chloride (PVC) industry. Compared with an organotin thermal stabilizer, the calcium-zinc thermal stabilizer is usually odorless, having a lower cost, thus it has become an important research field in a PVC additives industry. One research content of the calcium-zinc stabilizer is improving a thermal stability thereof to PVC at a same time: an initial coloring performance and a rapid aging ("zinc burning") during a later stage.

Chinese patent CN106633053A has disclosed a preparation and application of a novel PVC stabilizer of a cyanuric acid-uracil copolymer zinc salt, wherein the cyanuric acid-uracil copolymer zinc salt is synthesized by a wet method, a reaction is carried out in a solvent, while a large amount of waste water will be discharged during a producing process, and a subsequent filtration and drying step will further consume a lot of thermal energy. In addition, a particle size of a product synthesized according to the wet method is too large, when used as a PVC thermal stabilizer, it will affect a subsequent PVC processing technology.

Therefore, the current technology needs to be improved and developed.

### BRIEF SUMMARY OF THE DISCLOSURE

According to the defects in the prior art described above, the present application aims to provide a method for a solid-phase synthesis of a cyanuric acid-uracil complex zinc salt, in order to solve a plurality of problems in the prior art that during a synthesis process of the cyanuric acid-uracil complex zinc salt, including a large amount of waste water discharging, a high energy consumption, and a large particle size of the product produced.

The technical solution of the present application to solve the technical problems is as follows:
a method for a solid-phase synthesis of a cyanuric acid-6-amino-1,3-dimethyluracil complex zinc salt,
wherein comprising a plurality of following steps:

mixing cyanuric acid and zinc oxide under a shearing condition to conduct a reaction and obtain zinc cyanurate;
mixing the zinc cyanurate with 6-amino-1,3-dimethyluracil under a shearing condition to conduct a reaction to prepare cyanuric acid-uracil-6-amino-1,3-dimethyluracil complex zinc salt, wherein the step of mixing the cyanuric acid and the zinc oxide under the shearing condition to conduct the reaction to obtain the zinc cyanurate comprises:
   placing the cyanuric acid, the zinc oxide and a plurality of oily substances compatible with the PVC into a ball mill for a ball milling treatment, reacting and preparing the zinc cyanurate, and wherein the oily substances compatible with the PVC is an epoxidized soybean oil.

The method for the solid-phase synthesis of the cyanuric acid-6-amino-1,3-dimethyluracil complex zinc salt, wherein during the process of preparing the zinc cyanurate, stearic acid is further added to the ball mill, with a mass ratio between the stearic acid and the zinc oxide of 1:3-5.

The method for the solid-phase synthesis of the cyanuric acid-6-amino-1,3-dimethyluracil complex zinc salt, wherein the step of mixing the zinc cyanurate with uracil under the shearing condition to conduct the reaction to prepare the cyanuric acid-uracil complex zinc salt, comprising:
placing the zinc cyanurate, the uracil and a plurality of oily substances compatible with the PVC into a ball mill for a ball milling treatment, to form a coordinate covalent bond between the zinc cyanurate and the 6-amino-1 ,3-dimethyluracil before generating the zinc cyanurate.

The method for the solid-phase synthesis of the cyanuric acid-6-amino-1,3-dimethyluracil complex zinc salt, wherein a molar ratio of the cyanuric acid to the zinc oxide is 2:3-5.

The method for the solid-phase synthesis of the cyanuric acid-6-amino-1,3-dimethyluracil complex zinc salt, wherein a molar ratio of the cyanuric acid to the zinc oxide is 1:2.

The method for the solid-phase synthesis of the cyanuric acid-6-amino-1,3-dimethyluracil complex zinc salt, wherein a molar ratio of the uracil to the zinc cyanurate is 1:3-6.

The method for a solid-phase synthesis of a cyanuric acid-6-amino-1,3-dimethyluracil complex zinc salt, wherein a molar ratio of the uracil to the zinc cyanurate is 1:4.

The method for the solid-phase synthesis of the cyanuric acid-6-amino-1,3-dimethyluracil complex zinc salt, wherein the uracil is 6-amino-1 ,3-dimethyluracil, and a structural formula thereof is:

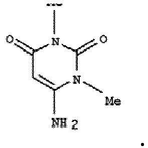

Benefits: the present application, by mixing the cyanuric acid and the zinc oxide under the shearing condition, conducts a reaction to obtain the zinc cyanurate; then by mixing the zinc cyanurate with the uracil under the shearing condition, conducts a reaction to prepare and obtain the cyanuric acid-uracil complex zinc salt. The present application adopts a method for a solid-phase synthesis of the cyanuric acid-uracil complex zinc salt, during a reaction process, there is no solvent required to disperse the reactants, thus no solvent to be treated upon the reaction completing, there is no waste water to be discharged, being clean and environmentally friendly, and a prepared reaction product of the cyanuric acid-uracil complex zinc salt has a small and evenly distributed particle size, being convenient to be used as a PVC thermal stabilizer, without affecting a subsequent processing of a PVC material.

### BRIEF DESCRIPTION OF THE DRAWINGS

FIG. 1 illustrates a flow chart on a method for the solid-phase synthesis of the cyanuric acid-uracil complex zinc salt in the present application;
FIG. 2 illustrates an infrared spectrum control chart in an infrared spectrum analysis for a sample of the cyanuric acid-uracil complex zinc salt as a product in an embodiment 2 and a directly mixed sample with a mass ratio of the zinc oxide: the cyanuric acid: the uracil = 8:6:1.

### DETAILED DESCRIPTION OF EMBODIMENTS

The present application provides a method for a solid-phase synthesis of a cyanuric acid-uracil complex zinc salt, in order to make the purpose, technical solution and the advantages of the present application clearer and more explicit, further detailed descriptions of the present application are stated herein, referencing to the attached drawings and some embodiments of the present application.

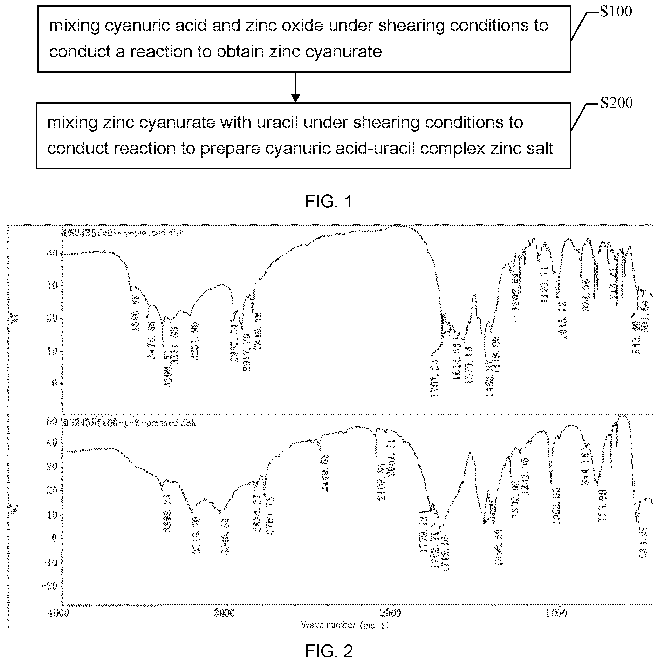

Referring to FIG.1, what is illustrated is a flow chart on an embodiment of a method for a solid-phase synthesis of a cyanuric acid-uracil complex zinc salt in the present application. Shown as FIG.1, the method comprises a plurality of steps:
S100, mixing cyanuric acid and zinc oxide under a shearing condition to conduct a reaction to obtain zinc cyanurate;
S200, mixing the zinc cyanurate with uracil under a shearing condition to conduct a reaction to prepare the cyanuric acid-uracil complex zinc salt.

A method for synthesizing the cyanuric acid-6-amino-1,3-dimethyluracil complex zinc salt in the prior art, adopting a wet synthesis, is carried out in an organic solvent, and during a reaction, it is required to add a dispersant and an initiator in an appropriate amount, thus having a preparation process complicated, and a large amount of waste water will be discharged during the reaction, while a particle size of the cyanuric acid-uracil complex zinc salt wet synthesized is too large, affecting a subsequent processing and application of the PVC.

The present embodiment adopts a solid-phase synthesis method to prepare the cyanuric acid-uracil copolymer zinc salt. During the reaction, no solvent is required to disperse the reactants. After the reaction is completed, there is no waste water to be treated, no waste water to be discharged, it is clean and environment-friendly. At a same time, a small amount of water produced during the production process may be processed by heating an equipment, having a preparation process simple, and an energy consumption small; and under the shearing condition, a product generated in the reaction of each group of reactants has a small particle size and a uniform particle size distribution, which is convenient to be used as a thermal stabilizer for the PVC, without affecting a subsequent processing of a PVC material.

In some embodiments, the cyanuric acid, the zinc oxide, and an epoxidized soybean oil are added to a ball mill for a ball milling, to react and produce zinc cyanurate. In the present embodiment, as an acidic nitrogen-containing compound, the cyanuric acid is easy to have a reaction with an amphoteric oxide (zinc oxide) to produce the zinc cyanurate and a small amount of water.

In the present embodiment, a reaction between the cyanuric acid and the zinc oxide is a solid-solid phase reaction. In order to increase a chance of contact and reaction between mixed raw materials (the cyanuric acid and the zinc oxide), the cyanuric acid and the zinc oxide are placed into the ball mill for a ball milling treatment. Since a gap between the ball mill and the mixed raw materials is small, the mixed raw materials can be changed into a very thin and wide form for the reaction through a ball milling process, which not only increases a reaction area of the mixed raw materials, but also increases a possibility of each reaction between elements, thus improves a reaction rate. Also, the ball mill keeps rotating during a running process, providing a large shear force, that can also increase a reaction rate as a whole.

In the present embodiment, a small amount of water may be generated during the reaction between the cyanuric acid and the zinc oxide, which can be evaporated by heating in the ball mill, while the water evaporated is non-toxic and harmless. In addition, although the oily substances compatible with the PVC added during the reaction are liquid, an amount is small and will be dispersed in the mixed raw materials. When the reaction is over, a small amount of filler is added, that is able to disperse and dry the product with a viscosity, so there is no waste water discharge problem exists in a whole reaction process.

In the present embodiment, during a process of ball milling the cyanuric acid and the zinc oxide, an epoxidized soybean oil is further added. The epoxidized soybean oil does not act as a solvent, instead it is mainly used to increase a viscosity between the mixed raw materials. If the mixed raw materials are too loose at first (low viscosity), it will be difficult to have a reaction in the ball mill, but after becoming sticky, a ball milling strength will be increased, improving a reaction rate between the mixed raw materials.

Since the cyanuric acid-6-amino-1,3-dimethyluracil complex zinc salt finally prepared in the present embodiment is applied as a thermal stabilizer for the PVC, and epoxidized soybean oil added to the ball mill will not be removed after the reaction is completed, the epoxidized soybean oil will be mixed among the cyanuric acid-6-amino-1,3-dimethyluracil complex zinc salts, a presence of the epoxidized soybean oil should not affect the subsequent processing of the PVC. Therefore, the epoxidized soybean oil having a better compatibility with the PVC is selected.

The epoxidized soybean oil is a chemical product made from a soybean oil after taking an oxidation treatment. It is a light yellow viscous oily liquid at a room temperature. It is a widely used non-toxic plasticizer and stabilizer for the PVC, having a good compatibility with the PVC, a low volatility and a low migration; having an excellent thermal stability and light stability, as well as a good water and oil resistance, being able to bring a product a good mechanical strength, weather resistance and electrical property, which is non-toxic and has been internationally recognized as a chemical process aid for a food packaging material. In the present embodiment, the epoxy soybean oil is selected to increase the viscosity of the mixed raw material, which can increase a reaction rate of the mixed raw material effectively during the ball milling process and maintain a stability of the reaction product.

In some embodiments, a molar ratio of the cyanuric acid to the zinc oxide is 2:3-5. Theoretically, the molar ratio of the reaction between the cyanuric acid and the zinc oxide is 2:3. In order to ensure that a zinc content of the cyanuric acid-6-amino-1,3-dimethyluracil complex zinc salt final synthesized can have an initial coloring effect in the stabilizer, an amount of the zinc oxide shall be more than what calculated theoretically. The inventor has obtained through a plurality of related experiments that the molar ratio of the cyanuric acid to the zinc oxide is preferred to be from 2:3 to 2:5. If the amount of the zinc oxide is too little, the zinc content of the zinc cyanuric acid-6-amino-1,3-dimethyluracil complex zinc salt final synthesized is too low to achieve an effect of improving an initial coloration in the stabilizer; if the zinc oxide is too much, the cyanuric acid-6-amino-1,3-dimethyluracil complex zinc salt final synthesized contains excess zinc oxide, resulting in a thermal stability performance of the PVC becoming worse, that is, a later zinc burning will be more serious. In some specific embodiments, the molar ratio of the cyanuric acid to the zinc oxide is 1:2.

In some embodiments, in the process of preparing the zinc cyanurate, a stearic acid is further added to the ball mill, and a mass ratio of the stearic acid to the zinc oxide is 1:3-5. Due to an amount of the zinc oxide added to a reaction system of the cyanuric acid and the zinc oxide is excessive, while a pure zinc oxide is not conducive to a long-term aging of the PVC, thus adding a small amount of stearic acid may combine with unreacted zinc oxide, a product thereof, Zinc stearate, is also a common stabilizer; further, the stearic acid has a lubricity, being able to better promote the reaction.

In some embodiments, the zinc cyanurate, uracil, and the epoxidized soybean oil are added to the ball mill for a ball milling, making the zinc cyanurate form a coordinate covalent bond with the uracil before forming the cyanuric acid-6-amino-1,3-dimethyluracil complex zinc salt. The present embodiment, wherein the reaction occurred between the zinc cyanurate and uracil is a complexation reaction. The zinc atom in the zinc cyanurate has an empty orbital and acts as a central atom, while the uracil contains imino groups, the atom has a lone electron pair and acts as a ligand. During the ball milling process of the zinc cyanurate and uracil, the lone electron pair on the imino group of the uracil can enter the empty orbital of the zinc atom in the zinc cyanurate, forming a coordinate covalent bond, before generating the zinc cyanurate-uracil complex zinc salt.

In the present embodiment, during the process of ball milling the zinc cyanurate and the uracil, epoxidized soybean oil is also added. The epoxidized soybean oil is mainly applied to increasing a viscosity between the zinc cyanurate and the uracil. If the zinc cyanurate and the uracil are too loose at first (low viscosity), it will be difficult to have a reaction in the ball mill, but after becoming sticky, a ball milling strength will be increased, improving a reaction rate between the zinc cyanurate and the uracil.

Since the cyanuric acid-6-amino-1,3-dimethyluracil complex zinc salt prepared in the present embodiment is applied as the thermal stabilizer for the PVC, and the epoxidized soybean oil added to the ball mill will not be removed after the reaction is completed, the epoxidized soybean oil will be mixed among the cyanuric acid-6-amino-1,3-dimethyluracil complex zinc salts, a presence of epoxidized soybean oil should not affect the subsequent processing of the PVC. Therefore, the epoxidized soybean oil having a better compatibility with the PVC is selected.

In some embodiments, a molar ratio of the uracil to the zinc cyanurate is 1 :3-6. A role of the uracil is taking a substitution reaction with allyl chloride, to improve an initial coloration ability. If an amount of the uracil is too much (a molar ratio of the uracil to the zinc cyanurate is greater than 1:3), a zinc content of the cyanuric acid-uracil complex zinc salt synthesized will be reduced, thus unable to achieve an effect of improving the initial coloration in the stabilizer; if the amount of the uracil is too little (the molar ratio of the uracil to the zinc cyanurate is less than 1:6), it will be difficult to form a complex. In some specific embodiments, the molar ratio of the uracil to the zinc cyanurate is 1:4.

The uracil is 6-amino-1,3-dimethyluracil, and a structural formula thereof is: 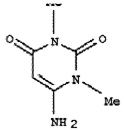 In the present embodiment, the 6-amino-1,3-dimethyluracil, in addition to a lone electron pair in the imino group in a benzene ring, a nitrogen in an amino group on the benzene ring has also a lone electron pair. Both lone electron pairs are able to enter the empty orbital of the zinc atom in the zinc cyanurate to form the coordinate covalent bond, before finally producing the zinc cyan urate-uracil complex zinc salt. Thus the 6-amino-1 ,3-dimethyluracil is more likely to undergo a complexation reaction with the zinc cyanurate, thereby increasing a yield of the product, and the zinc cyanurate-uracil complex zinc salt prepared has a more stable performance.

In some embodiments, during the ball milling process for the reactants, and after the reaction is complete, the epoxidized soybean oil will not be removed, while an existence of epoxidized soybean oil will cause an insufficient dispersion between a plurality of particles in the product. Therefore, by adding a small amount of filler to the product, the product with a certain viscosity can be dispersed, which is beneficial to the subsequent use as a thermal stabilizer.

Further detailed descriptions of the present application are stated hereafter, referencing to some embodiments of the present application.

### Embodiment 1

Weigh cyanuric acid, zinc oxide, and uracil in a mole number ratio of 6:9:1 with a ball-to-material ratio adopted as 30:1. First place the cyanuric acid and the zinc oxide in a ball mill, and add a small amount of the epoxidized soybean oil, adjust a speed of the ball mill to 300 r/min, and grind for 4 hours to obtain the zinc cyanurate; then add the uracil to the ball mill and continue to grind for 4 hours to obtain the cyanuric acid-uracil complexed zinc salt; and finally heat the ball mill to dry the cyanuric acid-uracil complexed zinc salt synthesized and add an appropriate amount of the dispersant, to obtain the product.

### Embodiment 2

Weigh zinc oxide, cyanuric acid and uracil in a mass ratio of 8:6:1 for use, and a ball-to-material ratio of 30:1 is adopted. First place the cyanuric acid and the zinc oxide in a ball mill, and add a small amount of the epoxidized soybean oil, adjust a speed of the ball mill to 300 r/min, and grind for 5 hours to obtain the zinc cyanurate; then add 6-amino-1 ,3- Dimethyluracil to the ball mill and grind for 4 hours to obtain the cyanuric acid-6-amino-1,3-dimethyluracil complex zinc salt; finally, the synthesized cyanuric acid-uracil complex zinc salt is dried by heating a ball mill and an appropriate amount of dispersant is added to obtain the product.

A sample of the zinc acid-6-amino-1,3-dimethyluracil complex zinc salt prepared in the embodiment 2 and a sample directly mixed according to a mass ratio of zinc oxide: cyanuric acid: uracil=8:6:1 are analyzed and compared by an infrared spectroscopy, an infrared contrast spectrum obtained is shown in FIG.2. As shown in FIG.2, an upper spectrum is the infrared spectrum of the sample in the embodiment 2, and a lower spectrum is the infrared spectrum of the sample after direct mixing. It can be seen from FIG.2 that the upper spectrum has obviously a plurality of peaks appear, which means that the sample synthesized in the embodiment 2 have a plurality of chemical bonds not contained in the mixed sample. That means by using the present method, the zinc oxide, the cyanide acid and the uracil have a chemical reaction occurred, a new chemical bond formed, and a new structure generated.

All above, the present application adopts a solid phase synthesis method to prepare the zinc acid-6-amino-1,3-dimethyluracil complex zinc salt, the reactant is dry-milled in a device with a high shear force and a high contact area to obtain the cyanuric acid-uracil complex zinc salt. The preparation method is simple and easy to operate. Water is produced during the reaction, but an amount of the water produced is very little, and it can be removed by simply heating the equipment, without requiring any other drying treatments with high energy consumption. In addition, there is no solvent required in the reaction process to disperse the reactants. After the reaction is completed, there is no solvent to be treated, no waste water to be discharged, it is clean and environmentally friendly. The reaction product of the cyanuric acid-uracil complex zinc salt is prepared by a dry milling, thus the product generated has a small particle size and a uniform particle size distribution, which is convenient for use as a PVC thermal stabilizer and will not affect a subsequent processing of a PVC materials.

It should be understood that, the application of the present application is not limited to the above examples listed. Ordinary technical personnel in this field can improve or change the applications according to the above descriptions, all of these improvements and transforms should belong to the scope of protection in the appended claims of the present application.

## Claims

1. A method for a solid-phase synthesis of a cyanuric acid-6-amino-1,3-dimethyluracil complex zinc salt, wherein comprising a plurality of following steps:
mixing cyanuric acid and zinc oxide under a shearing condition to conduct a reaction and obtain zinc cyanurate;
mixing the zinc cyanurate with uracil under a shearing condition to conduct a reaction to prepare cyanuric acid-uracil complex zinc salt; wherein the step of mixing the cyanuric acid and the zinc oxide under the shearing condition to conduct the reaction to obtain the zinc cyanurate comprises:
placing the cyanuric acid, the zinc oxide and a plurality of oily substances compatible with the PVC into a ball mill for a ball milling treatment, reacting and preparing the zinc cyanurate; and
wherein the oily substances compatible with the PVC is an epoxidized soybean oil.

2. The method for the solid-phase synthesis of the cyanuric acid-6-amino-1,3-dimethyluracil complex zinc salt according to claim 1, wherein during the process of preparing the zinc cyanurate, stearic acid is further added to the ball mill, with a mass ratio between the stearic acid and the zinc oxide of 1:3-5.

3. The method for the solid-phase synthesis of the cyanuric acid-6-amino-1,3-dimethyluracil complex zinc salt according to claim 1, wherein the step of mixing the zinc cyanurate with uracil under the shearing condition to conduct the reaction to prepare the cyanuric acid-uracil complex zinc salt, comprises:
placing the zinc cyanurate, the uracil and a plurality of oily substances compatible with the PVC into a ball mill for a ball milling treatment, to form a coordinate covalent bond between the zinc cyanurate and the 6-amino-1,3-dimethyluracil, before generating the zinc cyanurate complex salt.

4. The method for the solid-phase synthesis of the cyanuric acid-6-amino-1,3-dimethyluracil complex zinc salt according to any one of claims 1-3, wherein a molar ratio of the cyanuric acid to the zinc oxide is 2:3-5.

5. The method for the solid-phase synthesis of the cyanuric acid-6-amino-1,3-dimethyluracil complex zinc salt according to any one of claims 1-3, wherein a molar ratio of the cyanuric acid to the zinc oxide is 1:2.

6. The method for the solid-phase synthesis of the cyanuric acid-6-amino-1,3-dimethyluracil complex zinc salt according to any one of claims 1-3, wherein a molar ratio of the uracil to the zinc cyanurate is 1:3-6.

7. The method for the solid-phase synthesis of the cyanuric acid-6-amino-1,3-dimethyluracil complex zinc salt according to any one of claims 1-3, wherein a molar ratio of the uracil to the zinc cyanurate is 1:4.

## Patentansprüche

1. Verfahren zu einer Festphasensynthese eines Cyanursäure-6-Amino-1,3-Dimethyluracil-Komplex-Zinksalzes, umfassend eine Vielzahl der folgenden Schritte:
Mischen Cyanursäure und Zinkoxid unter einer Scherbedingung zur Durchführung einer Reaktion, um Zinkcyanurat zu erhalten;
Mischen des Zinkcyanurats mit Uracil unter einer Scherbedingung, um eine Reaktion zur Herstellung eines Cyanursäure-Uracil-Komplex-Zinksalzes durchzuführen; wobei der Schritt des Mischens der Cyanursäure und des Zinkoxids unter der Scherbedingung zur Durchführung einer Reaktion, um das Zinkcyanurat zu erhalten, umfasst:
Einbringen der Cyanursäure, des Zinkoxids und einer Vielzahl von mit dem PVC verträglichen öligen Substanzen in eine Kugelmühle für eine Kugelmahlbehandlung, Reaktion und Herstellung des Zinkcyanurats; und
wobei es sich bei den mit dem PVC verträglichen öligen Substanzen um ein epoxidiertes Sojabohnenöl handelt.

2. Verfahren zur Festphasensynthese des Cyanursäure-6-Amino-1,3-Dimethyluracil-Komplex-Zinksalzes nach Anspruch 1, wobei während des Verfahrens zur Herstellung des Zinkcyanurats weiterhin Stearinsäure in die Kugelmühle gegeben wird, mit einem Massenverhältnis zwischen der Stearinsäure und dem Zinkoxid von 1:3-5.

3. Verfahren zur Festphasensynthese des Cyanursäure-6-Amino-1,3-Dimethyluracil-Komplex-Zinksalzes nach Anspruch 1, wobei der Schritt des Mischens des Zinkcyanurats mit Uracil unter der Scherbedingung, um die Reaktion zur Herstellung des Cyanursäure-Uracil-Komplex-Zinksalzes durchzuführen, umfasst:
Einbringen des Zinkcyanurats, des Uracils und einer Vielzahl von öligen Substanzen, die mit dem PVC kompatibel sind, in eine Kugelmühle für eine Kugelmahlbehandlung, um eine koordinative kovalente Bindung zwischen dem Zinkcyanurat und dem 6-Amino-1,3-Dimethyluracil zu bilden, bevor das Zinkcyanuratkomplexsalz erzeugt wird.

4. Verfahren zur Festphasensynthese des Cyanursäure-6-Amino-1,3-Dimethyluracil-Komplex-Zinksalzes nach einem der Ansprüche 1 bis 3, wobei ein Stoffmengenverhältnis von der Cyanursäure zum Zinkoxid 2:3 bis 5 beträgt.

5. Verfahren zur Festphasensynthese des Cyanursäure-6-Amino-1,3-Dimethyluracil-Komplex-Zinksalzes nach einem der Ansprüche 1 bis 3, wobei ein Stoffmengenverhältnis von der Cyanursäure zum Zinkoxid 1:2 beträgt.

6. Verfahren zur Festphasensynthese des Cyanursäure-6-Amino-1,3-Dimethyluracil-Komplex-Zinksalzes nach einem der Ansprüche 1 bis 3, wobei ein Stoffmengenverhältnis vom Uracil zum Zinkcyanurat 1:3 bis 6 beträgt.

7. Verfahren zur Festphasensynthese des Cyanursäure-6-Amino-1,3-Dimethyluracil-Komplex-Zinksalzes nach einem der Ansprüche 1 bis 3, wobei ein Stoffmengenverhältnis vom Uracil zum Zinkcyanurat 1:4 beträgt.

## Revendications

1. Procédé de synthèse en phase solide d'un sel de zinc complexe d'acide cyanurique-6-amino-1,3-diméthyluracile, dans lequel il comprend une pluralité d'étapes suivantes consistant à :
mélanger de l'acide cyanurique et de l'oxyde de zinc dans une condition de cisaillement afin d'effectuer une réaction de manière à obtenir du cyanurate de zinc ; mélanger le cyanurate de zinc avec de l'uracile dans une condition de cisaillement afin d'effectuer une réaction de manière à préparer un sel de zinc complexe d'acide cyanurique-uracile ; dans lequel l'étape consistant à mélanger l'acide cyanurique et l'oxyde de zinc dans la condition de cisaillement afin d'effectuer la réaction de manière à obtenir du cyanurate de zinc comprend :
la mise de l'acide cyanurique, de l'oxyde de zinc et d'une pluralité de substances huileuses compatibles avec le PVC dans un broyeur à boulets pour un traitement de broyage à boulets, la réaction et la préparation du cyanurate de zinc ; et
dans lequel les substances huileuses compatibles avec le PVC sont une huile de soja époxydée.

2. Procédé de synthèse en phase solide du sel de zinc complexe d'acide cyanurique-6-amino-1,3-diméthyluracile selon la revendication 1, dans lequel, au cours du processus de préparation du cyanurate de zinc, de l'acide stéarique est en outre ajouté au broyeur à boulets, avec un rapport massique entre l'acide stéarique et l'oxyde de zinc de 1:3 à 5.

3. Procédé de synthèse en phase solide du sel de zinc complexe d'acide cyanurique-6-amino-1,3-diméthyluracile selon la revendication 1, dans lequel l'étape consistant à mélanger le cyanurate de zinc avec de l'uracile dans la condition de cisaillement afin d'effectuer la réaction de manière à préparer le sel de zinc complexe d'acide cyanurique-uracile, comprend :
la mise du cyanurate de zinc, de l'uracile et d'une pluralité de substances huileuses compatibles avec le PVC dans un broyeur à boulets pour un traitement de broyage à boulets, afin de former une liaison covalente de coordination entre le cyanurate de zinc et le 6-amino-1,3-diméthyluracile, avant de générer le sel complexe de cyanurate de zinc.

4. Procédé de synthèse en phase solide du sel de zinc complexe d'acide cyanurique-6-amino-1,3-diméthyluracile selon l'une quelconque des revendications 1 à 3, dans lequel un rapport molaire entre l'acide cyanurique et l'oxyde de zinc est de 2:3 à 5.

5. Procédé de synthèse en phase solide du sel de zinc complexe d'acide cyanurique-6-amino-1,3-diméthyluracile selon l'une quelconque des revendications 1 à 3, dans lequel un rapport molaire entre l'acide cyanurique et l'oxyde de zinc est de 1:2.

6. Procédé de synthèse en phase solide du sel de zinc complexe d'acide cyanurique-6-amino-1,3-diméthyluracile selon l'une quelconque des revendications 1 à 3, dans lequel un rapport molaire entre l'uracile et le cyanurate de zinc est de 1:3 à 6.

7. Procédé de synthèse en phase solide du sel de zinc complexe d'acide cyanurique-6-amino-1,3-diméthyluracile selon l'une quelconque des revendications 1 à 3, dans lequel un rapport molaire entre l'uracile et le cyanurate de zinc est de 1:4.
